# FASCICULE DE BREVET EUROPEEN

(11) **EP 3 094 415 B1**
(45) Date de publication et mention de la délivrance du brevet: **14.11.2018**
(21) Numéro de dépôt: 15704038.7
(22) Date de dépôt: 09.01.2015
(51) Int. Cl.: B05B 1/34, A61M 5/315, A61M 11/06, A61M 15/08, B05B 11/00, B05B 11/02

(54) **PROCEDE D'UTILISATION D'UN ENSEMBLE DE DISTRIBUTION DE PRODUIT FLUIDE**
VERFAHREN ZUR VERWENDUNG EINER ANORDNUNG FÜR DIE AUSGABE EINES FLÜSSIGPRODUKTS
METHOD FOR USING AN ASSEMBLY FOR DISPENSING A FLUID PRODUCT

(30) Priorité: 13.01.2014 FR 1450251
(43) Date de publication de la demande: 23.11.2016
(73) Titulaire: APTAR France SAS, 27110 Le Neubourg (FR)
(72) Inventeur: PETIT, Ludovic, F-27110 Vitot (FR); MARTINS, Olivier, F-27340 Les Damps (FR)
(74) Mandataire: CAPRI
(86) Numéro de dépôt international: PCT/FR2015/050050
(87) Numéro de publication internationale: WO 2015/104511

(56) Documents cités:
- EP-A1- 2 213 322
- FR-A3- 2 561 925
- JP-U- 3 047 521
- US-A- 3 874 380
- US-A- 4 767 416
- US-A- 4 962 868
- US-A- 5 961 489

## Description

La présente invention concerne un procédé d'utilisation d'un ensemble de distribution de produit fluide.

Les dispositifs de distribution de produit fluide sont bien connus. Ils comportent généralement un réservoir, des moyens de distribution et une tête de distribution. La tête de distribution peut être une tête de pulvérisation, par exemple du type nasale. Ces têtes de pulvérisation comportent généralement deux pièces, à savoir un corps formant la tête et une pièce formant le gicleur, c'est-à-dire la partie qui crée la pulvérisation en faisant tourbillonner le produit fluide lorsqu'il est distribué. Dans le cas d'un gicleur externe, un petit manchon vient s'emmancher par l'extérieur dans l'extrémité aval du corps pour définir avec celui-ci ledit profil de pulvérisation. Dans le cas d'un gicleur dit interne, c'est un insert inséré par l'intérieur de la tête qui vient coopérer avec la paroi d'extrémité du corps pour définir le profil de pulvérisation. Dans les deux cas, les profils de pulvérisation comprennent généralement une pluralité de canaux non radiaux, notamment trois, formés soit dans la paroi de fond de la tête, soit sur le manchon formant gicleur externe, soit sur l'insert formant gicleur interne. Généralement, les têtes de pulvérisation sont assemblées sur des tiges de pistons de pompes ou des soupapes de valves pour guider le produit, qui est distribué hors de la pompe ou de la valve, vers l'orifice de pulvérisation. Toutefois, il a aussi été proposé de disposer une tête de pulvérisation directement sur la sortie d'un réservoir de type seringue pour pulvériser le contenu de ladite seringue plutôt que de l'injecter à travers une aiguille. Le document WO 00/71263 décrit un tel arrangement. A nouveau, ces têtes de pulvérisation sont toujours réalisées avec deux ou plusieurs pièces assemblées les unes avec les autres. Cette mise en oeuvre empêche notamment l'utilisation de ces seringues en mode aspiration. En particulier, une seringue avec une tête de pulvérisation ne permet pas le mode aspiration typique des seringues, par exemple pour aspirer un produit fluide à partir d'un réservoir de prélèvement. Par ailleurs, une seringue avec une aiguille ne permet pas une pulvérisation du produit fluide lors de sa distribution.

Les documents US5961489, US4767416, US3874380, EP2213322, FR2561925 et US4962868 décrivent des dispositifs de l'art antérieur.

La présente invention a pour but de fournir un procédé d'utilisation d'un ensemble de distribution de produit fluide qui ne reproduit pas les inconvénients susmentionnés.

La présente invention a aussi pour but de fournir un procédé de distribution de produit fluide avec un ensemble de distribution de produit fluide, qui soit simple et peu coûteux à fabriquer et à assembler, et qui puisse fonctionner à la fois en mode aspiration et en mode expulsion.

La présente invention a également pour but de fournir un tel procédé de distribution de produit fluide qui permettant de réaliser une pulvérisation correcte et reproductible.

La présente invention a donc pour objet un procédé d'utilisation d'un ensemble de distribution de produit fluide tel que décrit dans la revendication 1. Des modes de réalisation avantageux sont décrits dans les revendications dépendantes.

Avantageusement, pour permettre d'évacuer la surpression générée dans ledit réservoir de mélange par l'injection dudit fluide, ledit réservoir et ledit piston coopèrent de manière non étanche en position totalement enfoncée du piston, pour relier l'intérieur du réservoir de mélange à l'atmosphère dans cette position totalement enfoncée du piston.

Selon l'invention, ladite tête de prélèvement est réalisée d'une pièce monobloc avec ledit réservoir, notamment par moulage d'un matériau plastique, et ladite tête de prélèvement comporte une aiguille de prélèvement.

Avantageusement, ladite tête de pulvérisation comporte un canal central d'expulsion se terminant, dans le sens d'écoulement du produit fluide lors de la pulvérisation, par un orifice de pulvérisation, un profil de pulvérisation étant prévu en amont dudit orifice de pulvérisation.

Avantageusement, ledit réservoir et/ou ledit piston comporte(nt) des moyens de fractionnement de dose pour séparer le produit fluide à distribuer contenu dans ledit réservoir en au moins deux doses destinées à être pulvérisées lors de plusieurs actionnements successifs dudit ensemble.

Avantageusement, lesdits moyens de fractionnement de dose comportent un ergot dudit piston coulissant dans une rainure dudit réservoir, ladite rainure comportant un épaulement pour bloquer ledit ergot après pulvérisation de la première dose de produit fluide.

Avantageusement, ledit réservoir et/ou ledit piston comporte(nt) des moyens d'accumulation d'énergie nécessitant l'application d'au moins une force prédéterminée pour permettre la pulvérisation dudit produit fluide à distribuer.

Avantageusement, lesdits moyens d'accumulation d'énergie comportent au moins un bossage coopérant avec ledit ergot dudit piston, ledit ergot pouvant passer au-delà desdits moyens d'accumulation d'énergie lorsqu'au moins ladite force prédéterminée est appliquée sur ledit piston.

Ces avantages et caractéristiques et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en section d'un ensemble de distribution selon une première variante de réalisation avantageuse de la présente invention, avec la tête de pulvérisation en position assemblée,
La figure 2 est une vue schématique de coté de l'ensemble de distribution représenté sur la figure 1,
Les figures 3 et 4 sont des vues schématiques de l'ensemble de distribution des figures 1 et 2 avec la tête de pulvérisation en position non-assemblée, lors d'une phase de prélèvement d'un fluide tel qu'un solvant à partir d'un réservoir de prélèvement,
Les figures 5 et 6 sont des vues schématiques similaires à celles des figures 3 et 4, lors de phases d'injection du fluide tel qu'un solvant dans un réservoir de mélange et d'aspiration du produit fluide à distribuer à partir dudit réservoir de mélange,

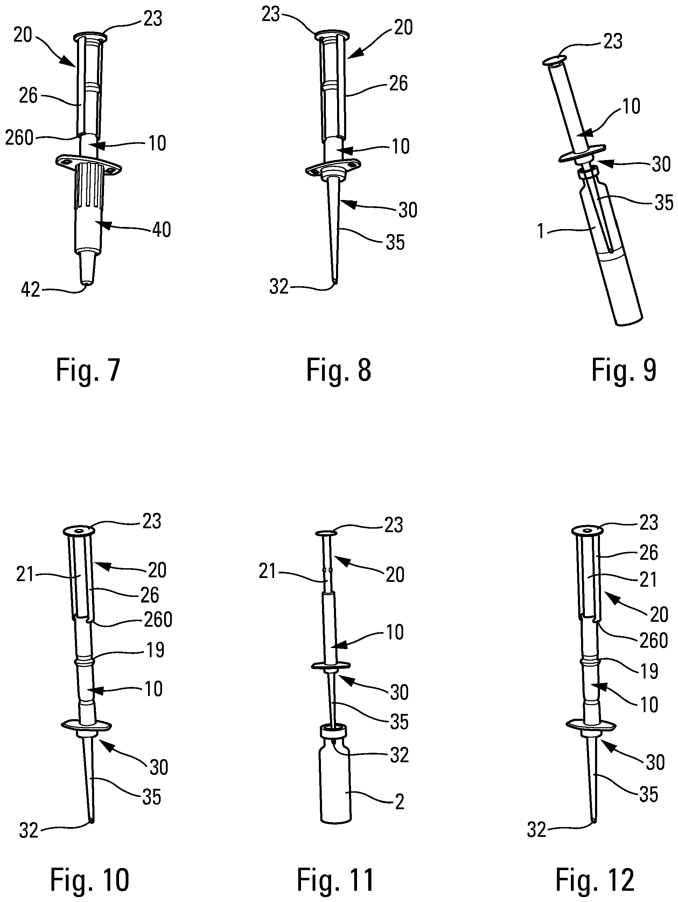

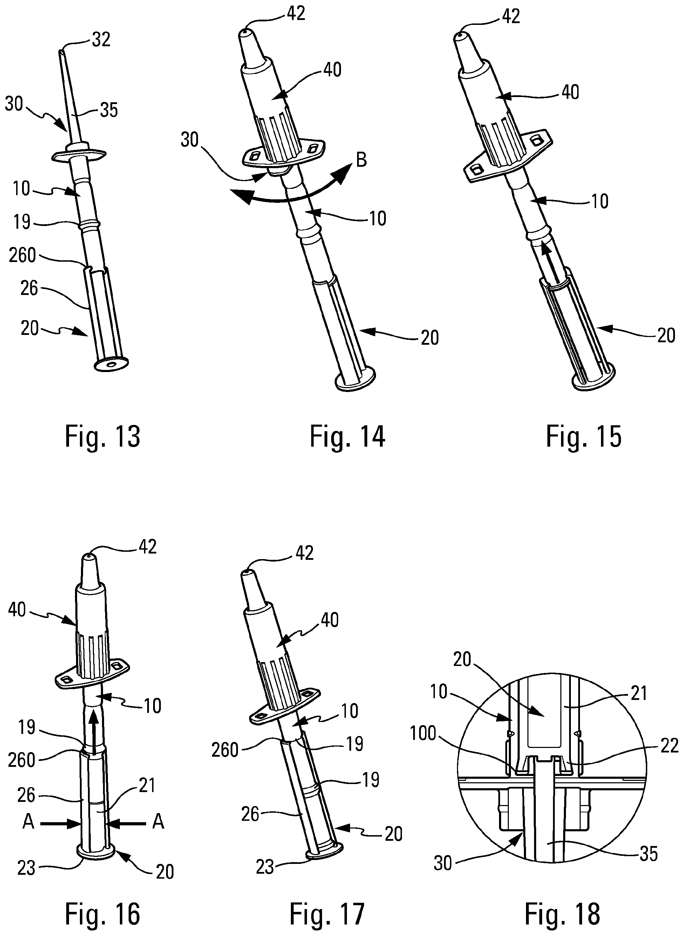

La figure 7 à 17 illustrent de manière très schématique un procédé d'utilisation d'un ensemble de distribution selon une seconde variante de réalisation avantageuse de la présente invention, la figure 9 illustrant une phase de prélèvement de solvant à partir d'un réservoir de prélèvement, la figure 11 illustrant une phase d'injection du solvant dans un réservoir de mélange, la figure 12 illustrant l'ensemble de distribution contenant le produit fluide à distribuer, les figures 13 et 14 illustrant l'assemblage de la tête de pulvérisation sur la tête de prélèvement, et les figures 15 à 17 illustrant les pulvérisations des deux demi-doses de produit fluide à distribuer, et
La figure 18 est une vue schématique de détail de l'éventation du réservoir de mélange réalisée en fin de phase d'injection du solvant.

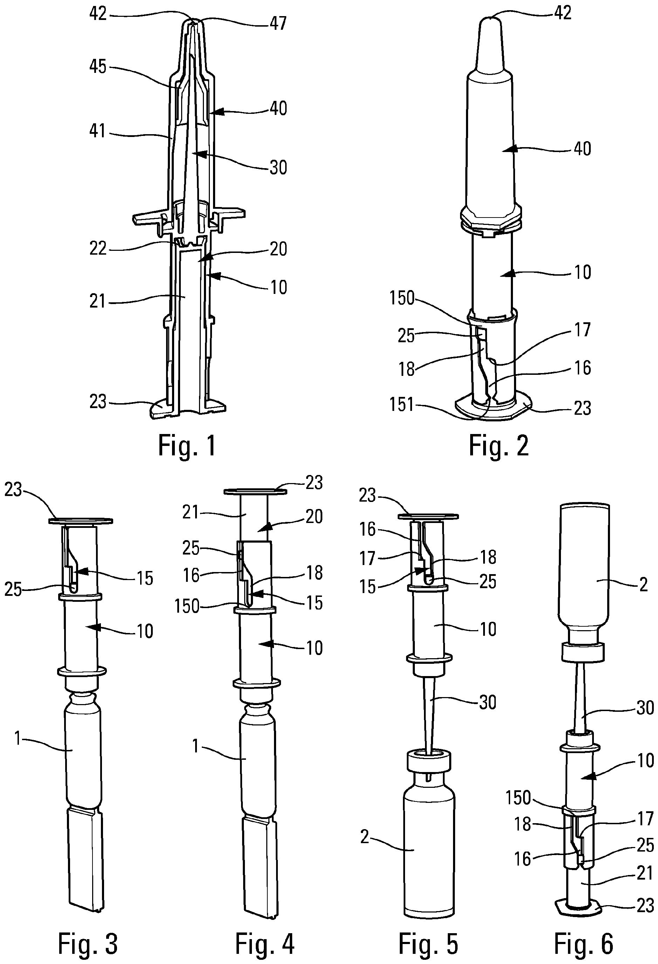

Les figures 1 à 6 représentent un ensemble de distribution selon une première variante de réalisation avantageuse de l'invention. Cet ensemble de distribution comporte un réservoir 10 du type seringue, un piston 20 coulissant dans ledit réservoir entre une position rétractée et une position enfoncée, une tête de prélèvement 30 monobloc avec le réservoir 10, et une tête de pulvérisation 40 qui vient s'assembler sur ladite tête de prélèvement. Les figures 1 et 2 montrent la tête de pulvérisation en position assemblée, et les figures 3 à 6 montrent ladite tête de pulvérisation en position non-assemblée.

Le réservoir 10 et la tête de prélèvement 30 sont de préférence réalisés par moulage d'un matériau plastique.

Le piston 20 comporte un élément de piston 22 solidaire d'une tige d'actionnement 21 actionnée par l'utilisateur au moment de la distribution, notamment en appuyant manuellement sur une collerette d'extrémité 23 de la tige de piston 21. L'élément de piston 22 peut être réalisé d'une pièce avec la tige de piston 21, par exemple par moulage d'un matériau plastique, ou former un élément séparé fixé, par exemple surmoulé, à ladite tige de piston. Dans ce cas, l'élément de piston 22 peut être réalisé en un matériau différent de la tige de piston 21.

Dans la variante des figures 1 à 6, le réservoir 10 comporte au moins une rainure 15, 16 qui coopère avec un ergot 25 de ladite tige de piston 21. Cet ergot 25 se projette radialement vers l'extérieur et coulisse dans ladite au moins une rainure 15 lors de l'actionnement dudit ensemble de distribution. Avantageusement, la rainure 15 comporte d'une part une butée finale 150 qui coopère avec ledit ergot 25 pour définir la position enfoncée du piston 20, et d'autre part au moins une projection latérale 151 qui coopère avec ledit ergot 25 pour définir la position rétractée du piston 20.

La rainure 15 peut comporter un épaulement 17 formant moyen de fractionnement de dose. Ainsi, lors de l'actionnement du piston 20 en vue de pulvériser le produit fluide à distribuer, l'ergot 25 du piston 20 coulisse axialement dans une première partie de rainure 16, et cet ergot 25 est stoppé par ledit épaulement 17 de la rainure 15. Ceci définit la première dose, en coupant la course d'actionnement du piston 20 dans le réservoir 10. L'utilisateur doit alors par exemple tourner légèrement le piston 20 pour amener l'ergot 25 en face d'une seconde partie de rainure 18 afin de pouvoir pulvériser la seconde dose. Bien entendu, l'invention s'applique aussi à un dispositif unidose, dans lequel la totalité du produit fluide à distribuer est pulvérisée en une seule dose. Elle peut aussi s'appliquer à des dispositifs contenant plus de deux doses, par exemple trois ou quatre doses.

Avantageusement, la rainure 15 comporte des moyens d'accumulation d'énergie, tels que par exemple des bossages, qui peuvent coopérer avec l'ergot 25 au début de chaque course d'actionnement pour imposer que l'utilisateur exerce au moins une force suffisante sur le piston 20 pour surmonter la résistance générée par lesdits moyens d'accumulation d'énergie. Lorsque cette force est atteinte, l'ergot 25 passe au-delà desdits moyens d'accumulation d'énergie, ce qui libère brusquement l'énergie accumulée dans la main de l'utilisateur, garantissant ainsi que la course d'actionnement, ou la demi-course d'actionnement dans l'exemple représenté d'un bidose, soit réalisée en totalité. Bien entendu, ces moyens d'accumulation d'énergie peuvent être réalisés d'une quelconque manière appropriée, et les bossages décrits ci-dessus ne sont qu'un exemple de réalisation avantageux. Par exemple on peut envisager des étranglements ou des ponts sécables dans ladite rainure 15.

Dans la variante des figures 7 à 17, qui illustrent une seconde variante de réalisation avantageuse de l'invention, les moyens de fractionnement de dose sont différents. Ils comportent ici un manchon externe fendu 26 du piston 20, ledit manchon fendu 26 s'étendant autour dudit réservoir 10. Le réservoir 10 comporte sur sa surface cylindrique radialement externe au moins un épaulement 19 qui va coopérer avec le bord d'extrémité axial 260 dudit manchon fendu 26 pour arrêter la course d'actionnement du piston 20 après pulvérisation de la première dose. Ceci est représenté sur la figure 16. Pour permettre la poursuite de la course d'actionnement du piston 20, l'utilisateur doit pincer latéralement le manchon fendu 26 du piston 20, dans le sens des flèches A sur la figure 16, ce qui va légèrement écarter radialement le bord d'extrémité axial 260 dudit manchon fendu 26, libérant ainsi le blocage du piston 20 par l'épaulement 19 du réservoir, et permettant ainsi de pulvériser la seconde dose de produit fluide.

D'autres variantes pour les moyens de fractionnement de dose sont envisageables, par exemple une partie élastiquement déformable formée soit sur le piston, soit sur le réservoir, et qui se déforme lorsqu'une force suffisante est appliquée sur le piston. Dans ce cas, les moyens de fractionnement de dose pourraient former simultanément moyens d'accumulation d'énergie. D'autres variantes sont aussi envisageables.

La tête de prélèvement 30 supporte une aiguille 35 pourvue d'un orifice 32. Cette aiguille 35 est donc avantageusement réalisée d'une pièce monobloc avec ledit réservoir 10, par exemple par moulage d'un matériau plastique. En variante, l'aiguille peut être fixée au réservoir d'une quelconque manière appropriée, par exemple par surmoulage. Dans ce cas, l'aiguille peut être réalisée en un matériau différent, par exemple du métal.

La tête de pulvérisation 40 comporte un corps creux 41 pourvu d'une paroi de fond 47 comportant un orifice de pulvérisation 42. Avantageusement, le corps creux 41 contient un insert 45 en amont de l'orifice de pulvérisation 42 pour s'adapter sur la tête du prélèvement 30. L'insert 45 comporte avantageusement un ou plusieurs passages latéraux (non représentés) qui permettent au produit fluide de passer de l'intérieur dudit insert 45 vers l'extérieur. La paroi d'extrémité axiale dudit insert 45 est alors fermée, et la surface externe de ladite paroi d'extrémité dudit insert peut former avec la paroi de fond 47 de la tête de pulvérisation 40, un profil de pulvérisation, comportant par exemple des canaux de tourbillonnement et une chambre de pulvérisation. Ce profil de pulvérisation permet de créer une bonne pulvérisation lors de la distribution du produit fluide à travers ladite tête de pulvérisation 40. Le profil de pulvérisation peut être formé sur la surface externe de ladite paroi d'extrémité dudit insert. En variante, le profil de pulvérisation peut être prévu dans la paroi de fond 47 de ladite tête de pulvérisation, auquel cas la surface externe de la paroi d'extrémité axiale de l'insert pourrait être lisse. De préférence, ladite tête de pulvérisation 40 et ledit insert 45 sont chacun réalisés par moulage d'un matériau plastique.

Les figures 3 à 6 illustrent partiellement les phases du procédé d'utilisation d'un ensemble de distribution selon la première variante de réalisation de la présente invention. Les figures 7 à 17 illustrent toutes les phases d'utilisation de l'ensemble de distribution selon la seconde variante de réalisation l'invention. Les différentes phases d'utilisation sont identiques dans ces deux exemples, seules les structures du piston 20 et du réservoir 10 diffèrent, comme expliqué ci-dessus.

Ainsi, les figures 3 et 4 d'une part et la figure 9 d'autre part montrent la phase de prélèvement, avec l'aiguille 35 de la tête de prélèvement 30 introduite dans un réservoir de prélèvement 1 contenant un fluide à aspirer dans le réservoir 10, notamment un solvant, tel que de l'eau. Ce prélèvement est réalisé de manière classique en faisant coulisser le piston 20 dans le réservoir 10 en éloignement de ladite aiguille 35, aspirant ainsi le fluide depuis ledit réservoir de prélèvement 1 dans ledit réservoir 10.

La figure 5 d'une part et la figure 11 d'autre part illustrent l'injection dudit fluide, qui a précédemment été prélevé depuis ledit réservoir de prélèvement 1, dans un réservoir de mélange 2 qui contient un autre produit, typiquement un produit actif, notamment sous forme de poudre ou de lyophilisat. Le mélange de cette poudre avec le solvant permet de reconstituer dans ledit réservoir de mélange 2 le produit fluide à distribuer. Avantageusement, le réservoir de mélange 2 peut être agité ou secoué pour favoriser ledit mélange. Avantageusement, pour permettre d'évacuer la surpression générée dans le réservoir de mélange 2 par l'injection du solvant, le réservoir 10 et le piston 20 sont réalisés de telle sorte à ne pas coopérer de manière étanche en position totalement enfoncée du piston 20, comme visible sur la figure 18, qui montre un dégagement radial 100 dans le fond du réservoir 10. Ceci permet de relier l'intérieur du réservoir de mélange 2 à l'atmosphère, via l'aiguille 35 et ledit passage d'air crée entre le piston 20 et le réservoir 10 en position enfoncée du piston 20. Ceci permet donc d'éviter tout risque de reconstitution incomplète dans le réservoir de mélange 2, ce qui pourrait se produire si celui-ci contenait une surpression.

Le produit fluide à distribuer, après reconstitution, est à nouveau aspiré dans le réservoir 10 à travers l'aiguille 35 de la tête de prélèvement, comme illustré schématiquement sur la figure 6.

La tête de pulvérisation 40 est alors assemblée autour de ladite tête de prélèvement 30, et le produit fluide à distribuer peut alors être pulvérisé à travers ladite tête de pulvérisation 40. Avantageusement, la tête de pulvérisation 40 est clipsée ou encliquetée sur le réservoir 10 et/ou sur la tête de prélèvement 30, par exemple par une rotation d'un quart de tour comme illustré par la flèche B sur la figure 14. Lors de cet assemblage, l'orifice 32 de l'aiguille 35 vient coopérer de manière sensiblement étanche dans ledit insert 45 de ladite tête de pulvérisation 40. Avantageusement, on peut prévoir que l'assemblage de la tête de pulvérisation 40 soit rendue indémontable, pour éviter une réutilisation de l'ensemble de distribution. Par ailleurs, la tête de pulvérisation 40 pourrait être préassemblée de manière amovible sur ladite tête de prélèvement 30 avant utilisation, l'utilisateur pouvant alors retirer ladite tête de pulvérisation 40, comme illustré par les figures 7 et 8, pour réaliser les phases de prélèvement, d'injection et d'aspiration du mélange reconstitué, et la remettre ensuite en position assemblée, pour réaliser les pulvérisations. En variante, l'ensemble de distribution pourrait être fourni avec la tête de pulvérisation 40 non assemblée avant utilisation.

Selon la variante principale de l'invention, le réservoir 10 de l'ensemble de distribution contient un premier produit, par exemple une poudre ou un lyophilisat, et le fluide, tel qu'un solvant, aspiré du réservoir de prélèvement 1 se mélange alors audit premier produit directement dans ledit réservoir 10 pendant la phase de prélèvement, sans qu'il ne soit nécessaire de passer par un réservoir de mélange.

Dans une autre variante, plusieurs fluides différents peuvent être aspirés à partir de plusieurs réservoirs de prélèvement différents, pour se mélanger dans le réservoir 10, avant pulvérisation.

Bien que la présente invention ait été décrite en référence à différentes variantes de réalisation avantageuses de celle-ci, il est entendu qu'un homme du métier peut y apporter toutes modifications utiles, sans sortir du cadre de la présente invention tel que défini par les revendications annexées.

## Revendications

1. Procédé d'utilisation d'un ensemble de distribution de produit fluide comportant un réservoir (10) destiné à contenir un produit fluide et un piston (20) coulissant dans ledit réservoir (10), ledit ensemble comportant une tête de prélèvement (30) solidaire dudit réservoir (10) pour aspirer un produit fluide à distribuer dans ledit réservoir (10), et une tête de pulvérisation (40) assemblée sur ladite tête de prélèvement (30) pour pulvériser le produit fluide à distribuer hors dudit réservoir (10), dans lequel ladite tête de prélèvement (30) est réalisée d'une pièce monobloc avec ledit réservoir (10), notamment par moulage d'un matériau plastique, et ladite tête de prélèvement (30) comporte une aiguille de prélèvement (35), ledit procédé comportant les étapes suivantes:
- aspirer un produit fluide à distribuer dans ledit réservoir (10) à travers ladite tête de prélèvement (30),
- assembler ladite tête de pulvérisation (40) sur ladite tête de prélèvement (30), et
- pulvériser le produit fluide à distribuer à travers ladite tête de pulvérisation (40),
**caractérisé en ce que** ladite étape d'aspirer un produit fluide à distribuer comprend une première étape de prélèvement d'un fluide, tel qu'un solvant, à partir d'un réservoir de prélèvement (1), et une seconde étape de mélange dudit fluide avec un premier produit, tel qu'une poudre ou un lyophilisat, pour former le produit fluide à distribuer.

2. Procédé selon la revendication 1, dans lequel ladite seconde étape de mélange comprend d'injecter ledit fluide, tel qu'un solvant, dans un réservoir de mélange (2) contenant ledit premier produit, de sorte que ledit premier produit et ledit fluide se mélangent dans ledit réservoir de mélange (2) pour former le produit fluide à distribuer, puis d'aspirer le produit fluide à distribuer dans ledit réservoir (10) à travers ladite tête de prélèvement (30).

3. Procédé selon la revendication 2, dans lequel, pour permettre d'évacuer la surpression générée dans ledit réservoir de mélange (2) par l'injection dudit fluide, ledit réservoir (10) et ledit piston (20) coopèrent de manière non étanche en position totalement enfoncée du piston (20), pour relier l'intérieur du réservoir de mélange (2) à l'atmosphère dans cette position totalement enfoncée du piston (20).

4. Procédé selon la revendication 1, dans lequel ladite tête de pulvérisation (40) comporte un canal central d'expulsion se terminant, dans le sens d'écoulement du produit fluide lors de la pulvérisation, par un orifice de pulvérisation (42), un profil de pulvérisation étant prévu en amont dudit orifice de pulvérisation.

5. Procédé selon les revendications 1 et/ou 4 dans lequel ledit réservoir (10) et/ou ledit piston (20) comporte(nt) des moyens de fractionnement de dose (15, 17 ; 25) pour séparer le produit fluide à distribuer contenu dans ledit réservoir (10) en au moins deux doses destinées à être pulvérisées lors de plusieurs actionnements successifs dudit ensemble.

6. Procédé selon la revendication 5, dans lequel lesdits moyens de fractionnement de dose comportent un ergot (25) dudit piston (20) coulissant dans une rainure (15) dudit réservoir (10), ladite rainure comportant un épaulement (17) pour bloquer ledit ergot (25) après pulvérisation de la première dose de produit fluide.

7. Procédé selon l'une quelconque des revendications 1 et/ou 4-6 dans lequel ledit réservoir (10) et/ou ledit piston (20) comporte(nt) des moyens d'accumulation d'énergie nécessitant l'application d'au moins une force prédéterminée pour permettre la pulvérisation dudit produit fluide à distribuer.

8. Procédé selon les revendications 6 et 7 dans lequel lesdits moyens d'accumulation d'énergie comportent au moins un bossage coopérant avec ledit ergot (25) dudit piston, ledit ergot (25) pouvant passer au-delà desdits moyens d'accumulation d'énergie lorsqu'au moins ladite force prédéterminée est appliquée sur ledit piston (20).

## Patentansprüche

1. Verfahren zur Verwendung eines Ausgabeaufbaus für ein fluides Produkt, umfassend einen Behälter (10) zur Aufnahme eines fluiden Produkts und einen Kolben (20), der in dem Behälter (10) gleitet, wobei der Aufbau einen Entnahmekopf (30) in einem Stück mit dem Behälter (10) umfasst, um ein fluides Produkt zur Ausgabe in den Behälter (10) zu saugen, und einen Sprühkopf (40), der auf dem Entnahmekopf (30) montiert ist, um das fluide Produkt zur Ausgabe aus dem Behälter (10) zu versprühen, wobei der Entnahmekopf (30), insbesondere durch Formen eines Kunststoffmaterials, aus einem Stück mit dem Behälter (10) gebildet ist und wobei der Entnahmekopf (30) eine Entnahmenadel (35) umfasst, wobei das Verfahren die folgenden Schritte umfasst:
- Ansaugen eines fluiden Produkts zur Ausgabe durch den Entnahmekopf (30) in den Behälter (10),
- Montieren des Sprühkopfes (40) auf dem Entnahmekopf (30), und
- Versprühen des fluiden Produkts zur Ausgabe durch den Sprühkopf (40),
**dadurch gekennzeichnet, dass** der Schritt des Ansaugens eines fluiden Produkts zur Ausgabe einen ersten Schritt der Entnahme eines Fluids, wie eines Lösungsmittels, aus einem Entnahmebehälter (1) und einen zweiten Schritt des Mischens des Fluids mit einem ersten Produkt, wie einem Pulver oder einem Lyophilisat, um das fluide Produkt zur Ausgabe zu bilden, umfasst.

2. Verfahren nach Anspruch 1, wobei der zweite Schritt des Mischens das Einspritzen des Fluids, wie eines Lösungsmittels, in einen Mischbehälter (2), der das erste Produkt enthält, derart dass sich das erste Produkt und das Fluid in dem Mischbehälter (2) vermischen, um das fluide Produkt zur Ausgabe zu bilden, und dann das Ansaugen des fluiden Produkts zur Ausgabe in den Behälter (10) durch den Entnahmekopf (30) umfasst.

3. Verfahren nach Anspruch 2, wobei, um ein Abführen des in dem Mischbehälter (2) durch Einspritzen des Fluids erzeugten Überdrucks zu ermöglichen, der Behälter (10) und der Kolben (20) in der vollständig eingefahrenen Position des Kolbens (20) nicht dichtend zusammenwirken, um das Innere des Mischbehälters (2) in dieser vollständig eingefahrenen Position des Kolbens (20) mit der Atmosphäre zu verbinden.

4. Verfahren nach Anspruch 1, wobei der Sprühkopf (40) einen mittleren Austreibkanal umfasst, der in Fließrichtung des fluiden Produkts beim Versprühen in einer Sprühöffnung (42) mündet, wobei ein Sprühprofil stromaufwärts der Sprühöffnung vorgesehen ist.

5. Verfahren nach den Ansprüchen 1 und/oder 4, wobei der Behälter (10) und/oder der Kolben (20) Dosisteilmittel (15, 17; 25) zum Trennen des fluiden Produkts zur Ausgabe, das in dem Behälter (10) enthalten ist, in mindestens zwei Dosen, die bei mehreren aufeinanderfolgenden Betätigungen des Aufbaus versprüht werden sollen, umfasst.

6. Verfahren nach Anspruch 5, wobei die Dosisteilmittel einen Vorsprung (25) des Kolbens (20) umfassen, der in einer Nut (15) des Behälters (10) gleitet, wobei die Nut einen Absatz (17) umfasst, um den Vorsprung (25) nach dem Versprühen der ersten Dosis des fluiden Produkts zu blockieren.

7. Verfahren nach einem der Ansprüche 1 und/oder 4-6, wobei der Behälter (10) und/oder der Kolben (20) Energiespeichermittel umfasst (umfassen), die das Aufbringen mindestens einer vorbestimmten Kraft erfordern, um das Versprühen des fluiden Produkts zur Ausgabe zu ermöglichen.

8. Verfahren nach den Ansprüchen 6 und 7, wobei die Energiespeichermittel mindestens einen Umfangswulst umfassen, der mit dem Vorsprung (25) des Kolbens zusammenwirkt, wobei der Vorsprung (25) über die Energiespeichermittel hinaus gehen kann, wenn zumindest die vorbestimmte Kraft auf den Kolben (20) aufgebracht wird.

## Claims

1. A method of using a fluid dispenser unit comprising a reservoir (10) for containing a fluid, and a piston (20) that slides in said reservoir (10), said unit further comprising an extractor head (30) that is secured to said reservoir (10) so as to suck a fluid to be dispensed into said reservoir (10), and a spray head (40) that is assembled on said extractor head (30) so as to spray the fluid to be dispensed out from said reservoir (10),
wherein said extractor head (30) is made as a single piece integrally with said reservoir (10), in particular by molding of a plastic material, and said extractor head (30) includes an extractor needle (35), said method comprising the following steps:
• sucking a fluid to be dispensed into said reservoir (10) through said extractor head (30);
• assembling said spray head (40) on said extractor head (30); and
• spraying the fluid to be dispensed through said spray head (40);
the method being **characterized in that** said step of sucking up a fluid to be dispensed comprises a first step of extracting a fluid, such as a solvent, from an extraction reservoir (1), and a second step of mixing said fluid with a first substance, such as a powder or a lyophilisate, so as to form the fluid to be dispensed.

2. A method according to claim 1, wherein said second step of mixing comprises: injecting said fluid, such as a solvent, into a mixing reservoir (2) containing said first substance, so that said first substance and said fluid mix together in said mixing reservoir (2) so as to form the fluid to be dispensed; then sucking the fluid to be dispensed into said reservoir (10) through said extractor head (30).

3. A method according to claim 2, wherein, in order to make it possible to evacuate the increased pressure generated in said mixing reservoir (2) by injecting said fluid, said reservoir (10) and said piston (20) co-operate in non-airtight manner in the fully depressed position of the piston (20), so as to connect the inside of the mixing reservoir (2) to the atmosphere in this fully depressed position of the piston (20).

4. A method according to claim 1, wherein said spray head (40) includes a central expulsion channel that, in the flow direction of the fluid during spraying, is terminated by a spray orifice (42), a spray profile being provided upstream of said spray orifice.

5. A method according to claims 1 and/or 4, wherein said reservoir (10) and/or said piston (20) include(s) dose-fractioning means (15, 17; 25) for dividing the fluid for dispensing that is contained in said reservoir (10) into at least two doses for being sprayed during a plurality of successive actuations of said unit.

6. A method according to claim 5, wherein said dose-fractioning means comprise a lug (25) of said piston (20) that slides in a groove (15) of said reservoir (10), said groove including a shoulder (17) for blocking said lug (25) after the first dose of fluid has been sprayed.

7. A method according to any one of claims 1 and/or 4-6, wherein said reservoir (10) and/or said piston (20) include(s) energy accumulation means that require at least a predetermined force to be applied in order to make it possible to spray said fluid to be dispensed.

8. A method according to claim 6 and claim 7, wherein said energy accumulation means comprise at least one projection that co-operates with said lug (25) of said piston, said lug (25) being able to pass beyond said energy accumulation means when at least said predetermined force is applied on said piston (20).
